# EUROPEAN PATENT SPECIFICATION

(11) **EP 2 685 994 B1**
(45) Date of publication and mention of the grant of the patent: **30.12.2015**
(21) Application number: 11719626.1
(22) Date of filing: 17.03.2011
(51) Int. Cl.: A61K 35/74

(54) **PROBIOTIC BACTERIA HAVING ANTIOXIDANT ACTIVITY AND USE THEREOF**
PROBIOTISCHEN BAKTERIEN MIT ANTIOXIDATIVER WIRKUNG UND IHRE VERWENDUNG
BACTÉRIES PROBIOTIQUES AYANT UNE ACTIVITÉ ANTIOXYDANTE ET LEUR UTILISATION

(43) Date of publication of application: 22.01.2014
(73) Proprietor: Probiotical S.p.A., 28100 Novara (IT)
(72) Inventor: MOGNA, Giovanni, I-28100 Novara NO (IT); STROZZI, Gian Paolo, I-28100 Novara NO (IT); MOGNA, Luca, I-28100 Novara NO (IT)
(74) Representative: Hoffmann Eitle
(86) International application number: PCT/IB2011/000561
(87) International publication number: WO 2012/123770

(56) References cited:
- WO-A2-2010/103374
- US-A1- 2006 233 774
- ZANONI S ET AL: "Growth kinetics on oligo- and polysaccharides and promising features of three antioxidative potential probiotic strains", JOURNAL OF APPLIED MICROBIOLOGY, OXFORD, GB, vol. 105, no. 5, 1 November 2008 (2008-11-01), pages 1266-1276, XP002597289, ISSN: 1364-5072
- MEEI-YN LIN ET AL: "Antioxidative Effect of Intestinal Bacteria Bifidobacterium longum ATCC 15708 and Lactobacillus acidophilus ATCC 4356", DIGESTIVE DISEASES AND SCIENCES, KLUWER ACADEMIC PUBLISHERS-PLENUM PUBLISHERS, NE, vol. 45, no. 8, 1 August 2000 (2000-08-01) , pages 1617-1622, XP019236955, ISSN: 1573-2568, DOI: 10.1023/A:1005577330695
- LIN M Y ET AL: "Inhibition of lipid peroxidation by Lactobacillus acidophilus and Bifidobacterium longum.", JOURNAL OF AGRICULTURAL AND FOOD CHEMISTRY SEP 1999 LNKD- PUBMED:10552700, vol. 47, no. 9, September 1999 (1999-09), pages 3661-3664, XP002665137, ISSN: 0021-8561
- PERAN L ET AL: "A comparative study of the preventative effects exerted by three probiotics, Bifidobacterium lactis, Lactobacillus casei and Lactobacillus acidophilus, in the TNBS model of rat colitis.", JOURNAL OF APPLIED MICROBIOLOGY OCT 2007 LNKD- PUBMED:17897185, vol. 103, no. 4, October 2007 (2007-10), pages 836-844, XP002665138, ISSN: 1364-5072

## Description

The present invention relates to certain bacterial strains for use in treating oxidative stress.

It is well known that all forms of life maintain a reducing environment within their cells. Any alteration in the redox state may cause toxic effects due to the production, and subsequent accumulation, of peroxides and free radicals. It is solely the excess thereof which is implicated in the oxidative stress that seems to be associated with many human pathologies, such as: atherosclerosis, arterial hypertension, Parkinson's disease, Alzheimer's disease, diabetes mellitus, colitis, rheumatoid arthritis.

In physiological conditions, an equilibrium exists between the levels of free radicals produced during normal cell metabolism and the levels of endogenous antioxidants, which are capable of protecting tissues from oxidative damage. The breaking of this equilibrium, due both to an increase in the production of radicals and a decrease in the levels of antioxidants, produces as a consequence the beginning of alterations in the structure and function of our cells. A cell physiologically possesses the capacity to perform antioxidant and hence protective action against free radicals thanks to the presence of specific defence mechanisms, both of an enzymatic and non-enzymatic nature.

Even though our cells possess a defence capacity, many factors of our everyday life contribute to diminishing it.

It is well-known, for example, that tobacco smoke and the intake of alcohol and drugs, as well as an excessive uncontrolled exposure to ionizing radiation can contribute to reducing the defence capacity of cells.

Moreover, the pace of everyday life, combined with an unbalanced diet containing little fruit, vegetables and fish, certainly does not enable our body to receive adequate supplementation of vitamins, minerals and trace elements having high antioxidant activity. Therefore, it is necessary to supplement the diet with a quantity of specific antioxidant substances that are really able to perform an antioxidant activity inside the human body.

In particular, it is necessary to have a composition which, once introduced into the body, is capable of supplementing the quantity of antioxidant substances normally present in the body, in such a way as to contribute to reducing oxidative stress.

Finally, it is necessary to have a composition which is effectively capable of maintaining antioxidant defences high after oxidative stress has been induced by external factors, for example after the intake of drugs.

The subject matter of the present invention relates to such compositions as set forth in the appended claims comprising certain probiotic bacteria for use in the treatment of oxidative stress.

Other preferred embodiments of the present invention are described below in the description and will be claimed in the appended dependent claims.

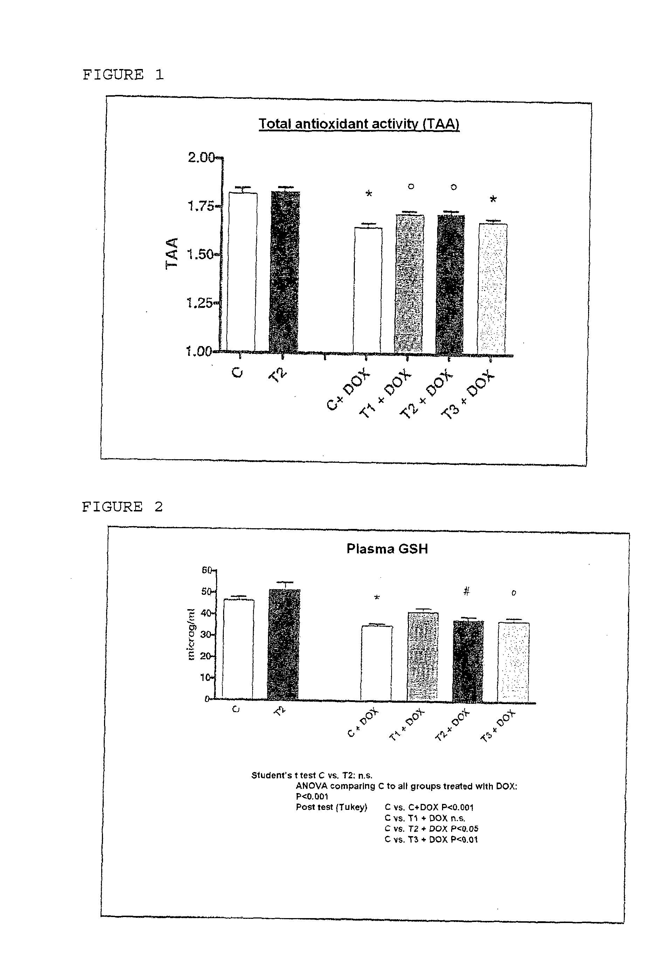

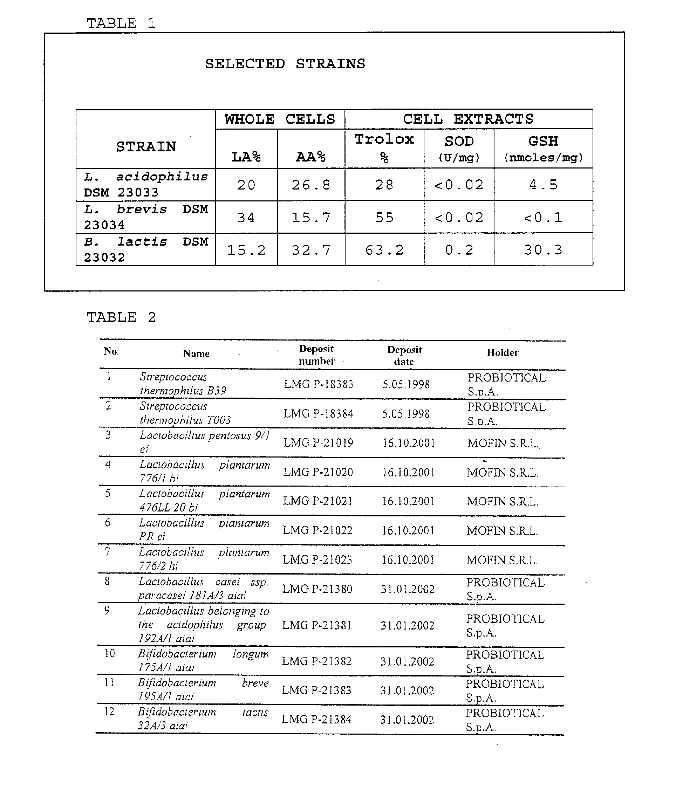

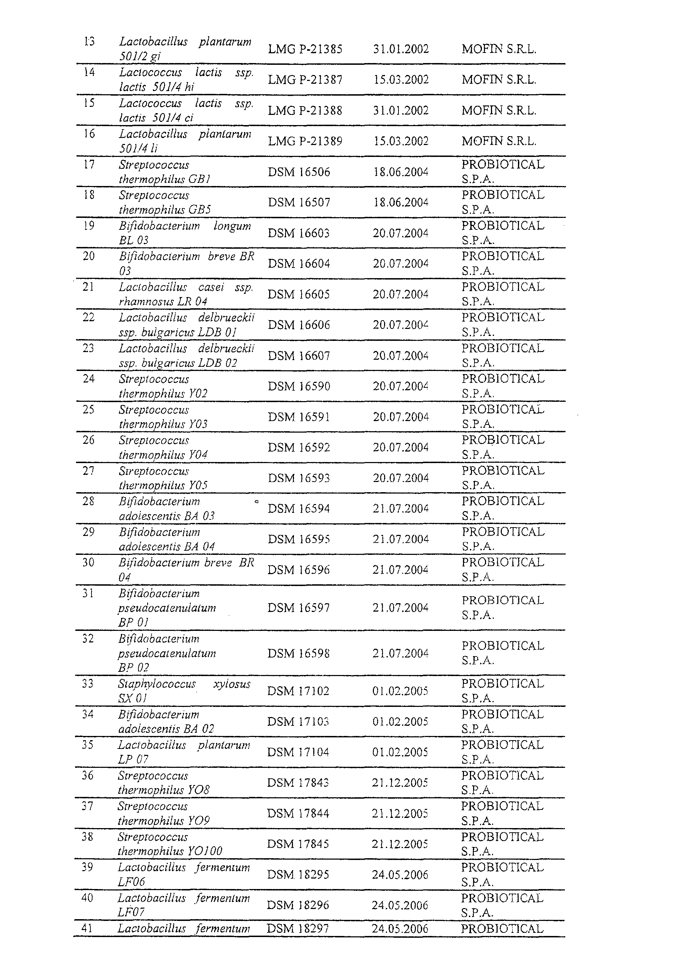

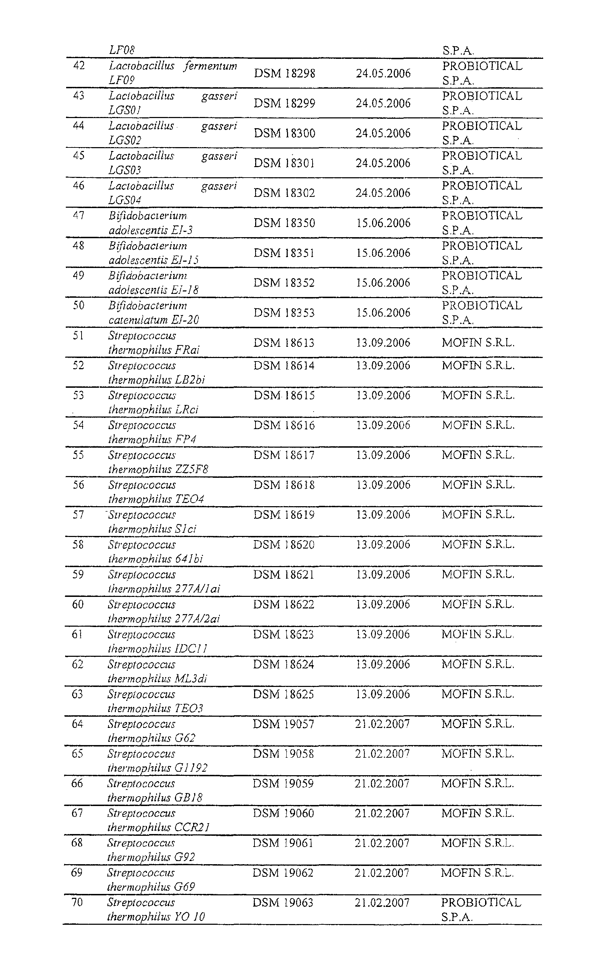

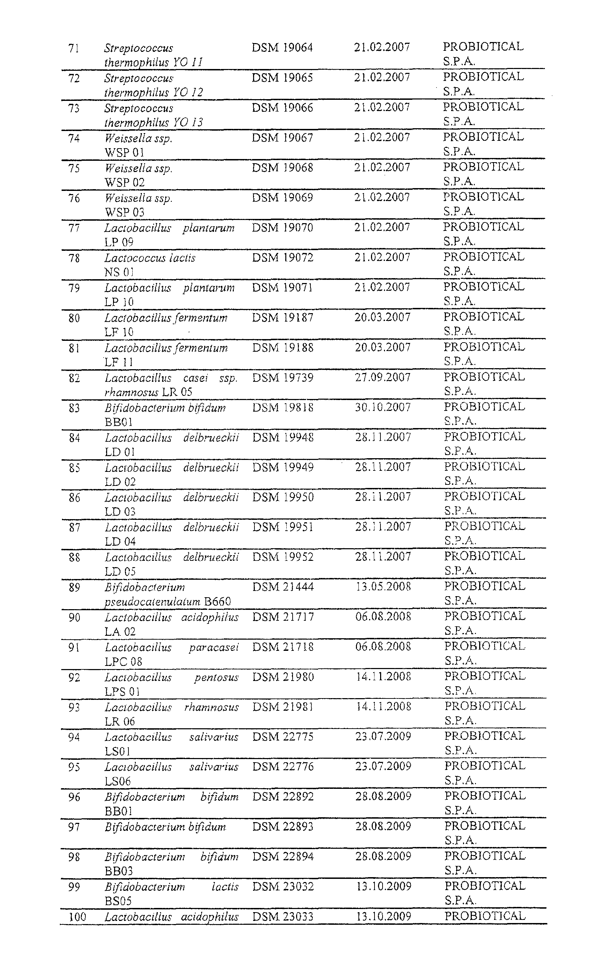

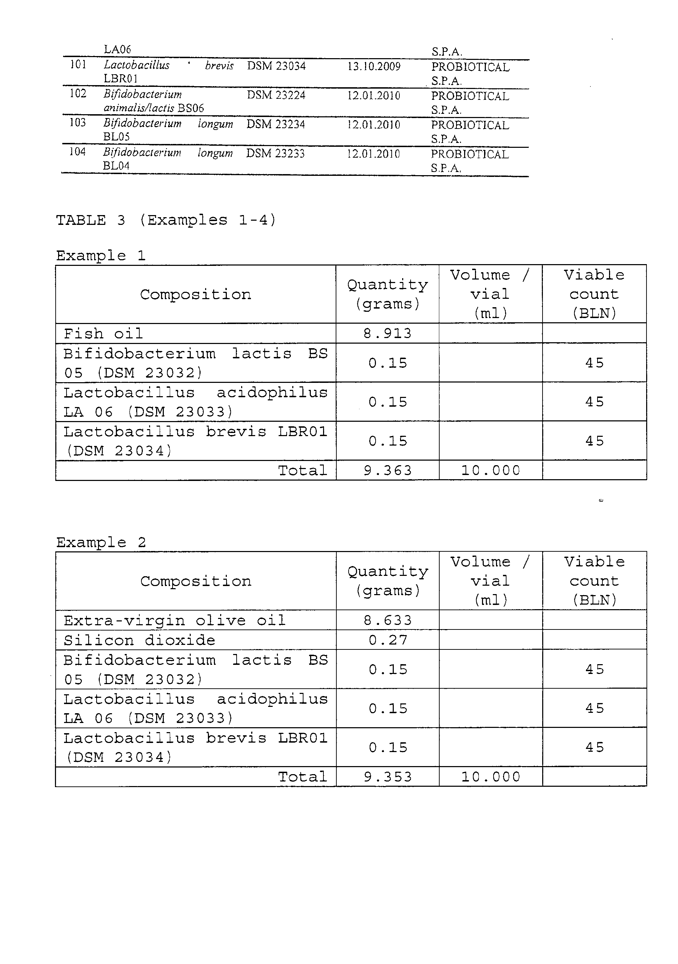

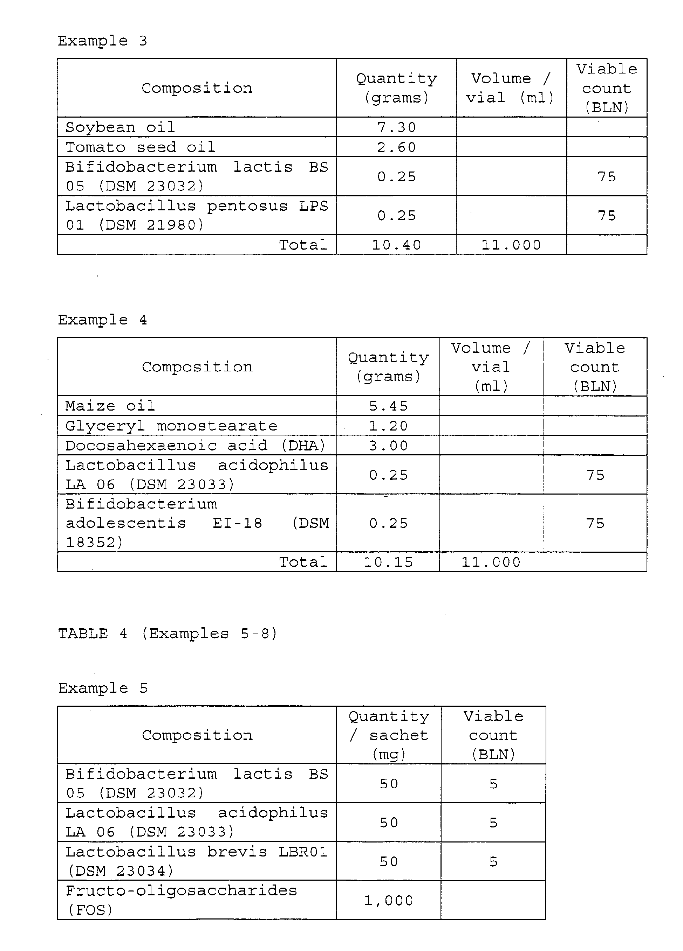

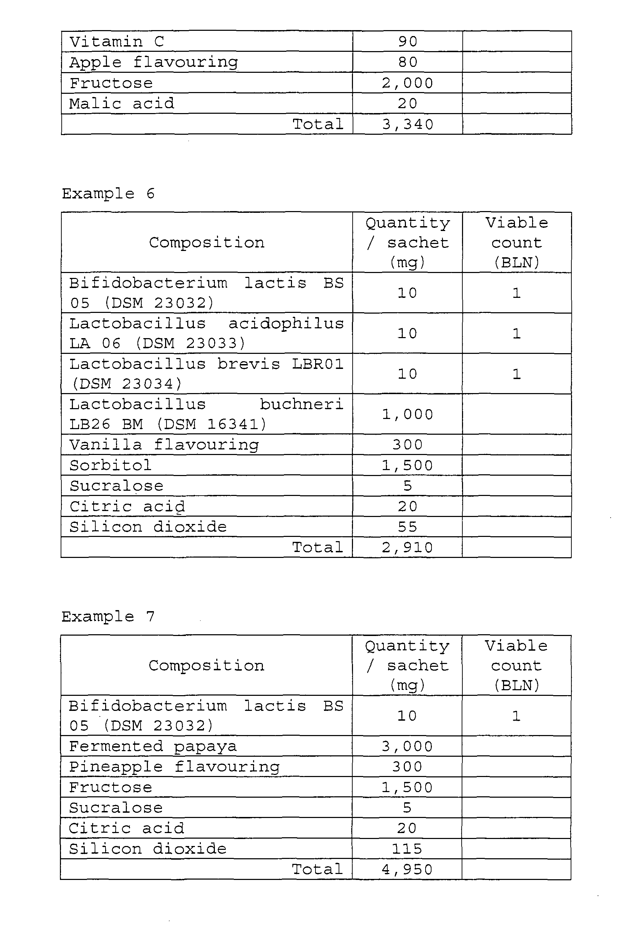

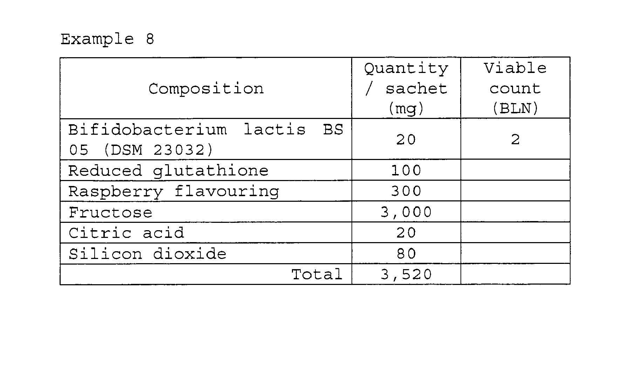

Figure 1 refers to a histogram that shows the values of total antioxidant activity (TAA) with respect to:
   - C (control sample under baseline conditions),
   - T2 (control sample treated with the probiotic bacteria of the present invention at the dose of 10⁸/day under baseline conditions),
   - C+DOX (control sample treated with Doxorubicin),
   - T1+DOX (sample treated with the probiotic bacteria of the present invention at the dose of 10⁹/day and Doxorubicin),
   - T2+DOX (sample treated with the probiotic bacteria of the present invention at the dose of 10⁸/day and Doxorubicin), and
   - T3+DOX (sample treated with the probiotic bacteria of the present invention at the dose of 10⁷/day and Doxorubicin).
Figure 2 refers to a histogram which shows the concentration values of plasma Glutathione (the antioxidant form of Glutathione is referred to as reduced Glutathione, GSH), as an evaluation of antioxidant capacity, with respect to:
   - C (control sample under baseline conditions),
   - T2 (control sample treated with the probiotic bacteria of the present invention at the dose of 10⁸/day under baseline conditions),
   - C+DOX (sample treated with Doxorubicin),
   - T1+DOX (sample treated with the probiotic bacteria of the present invention at the dose of 10⁹/day and Doxorubicin),
   - T2+DOX (sample treated with the probiotic bacteria of the present invention at the dose of 10⁸/day and Doxorubicin), and
   - T3+DOX (sample treated with the probiotic bacteria of the present invention at the dose of 10⁷/day and Doxorubicin).

Table 1 shows, by way of example, a group of microorganisms that have valid application in the context of the present invention.

The Applicant conducted intense research activity, after which it found that the bacterial strains belonging to a species selected from the group comprising *Lactobacillus acidophilus, Lactobacillus brevis* and *Bifidobacterium lactis* show a significant antioxidant activity, thanks to which it is possible to use the selected strains in a composition for use as a medication to reduce oxidative stress.

Advantageously, the composition of the present invention has application in cases in which the oxidative stress is induced as a result of the intake of drugs by a subject who is undergoing medical treatments.

In a preferred embodiment, the composition of the present invention can comprise a mixture of strains which contain one or more bacterial strains belonging to the species *Lactobacillus acidophilus,* for example two or three strains; one or more bacterial strains belonging to the species *Lactobacillus brevis,* for example two or three strains; and one or more bacterial strains belonging to the species *Bifidobacterium lactis,* for example two or three strains.

In a preferred embodiment, the composition of the present invention comprises a mixture containing at least one strain selected from the group comprising:
- *Bifidobacterium lactis* BS 05 (ID 1666) deposited by Probiotical SpA, Novara (Italy) with the DSMZ in Germany on 13.10.2009 and having the deposit number DSM 23032, and/or
- *Lactobacillus acidophilus* LA 06 (ID 1683) deposited by Probiotical SpA Novara (Italy) with the DSMZ in Germany on 13.10.2009 and having the deposit number DSM 23033, and/or
- *Lactobacillus brevis* LBR01 (ID 1685) deposited by Probiotical SpA, Novara (Italy) with the DSMZ in Germany on 13.10.2009 and having the deposit number DSM 23034.

Advantageously, the mixture of bacterial strains consists of *Bifidobacterium lactis* BS 05 (ID 1666) DSM 23032, *Lactobacillus acidophilus* LA 06 (ID 1683) DSM 23033 and *Lactobacillus brevis* LBR01 (ID 1685) DSM 23034.

In the context of the present invention, the bacterial strains can be in the form of live bacteria or dead bacteria or cellular components thereof, cell extracts and/or inactivated, lysed or permeabilized bacteria. The composition of the present invention can be a food composition, for example a symbiotic composition, or else a supplement or a pharmaceutical composition.

In a preferred embodiment, the composition can further comprise one or more bacterial strains among those listed in Table 2.

Preferably, the composition can moreover comprise from one to six strains, even more preferably from one to three strains selected from among those listed in Table 2.

Particularly preferred strains are selected from among those listed in Table 2, identified with the number present in the column on the left: No. 5, No. 20, No. 42, No. 49, No. 80, No. 81, No. 92, No. 93, No. 99, No. 100, and No. 101.

Strains No. 99, No. 100, and No. 101 are particularly preferred since they are endowed with a marked antioxidant activity.

Strains NO.5 and No. 20 are also endowed with antiinflammatory properties, among other things.

Strains No. 42, No. 80 and No. 81 are also capable of combating infections, e.g. intestinal yeast infections, including *Candida* infections.

Strain No. 49 is also capable of producing folates in the intestine.

Strains No. 5, No. 92 and No. 93 are also capable of antagonizing intestinal *E*. *coli.*

All of the strains described and/or claimed in the present patent application have been deposited in accordance with the Budapest Treaty and are made accessible to the public, on request, by the competent Depositing Authority.

Advantageously, the composition of the present invention can comprise elements or substances with antioxidant activity, such as, for example, selenium, zinc, magnesium, manganese, glutathione, superoxide dismutase (SOD), vitamin C, vitamin E, beta-carotene, carotenoids, riboflavin, taurine, L-carnosine, astaxanthin, lycopene, tomato seed oil, quercetin, tyrosol, resveratrol, hydroxytyrosol, oleuropein, lutein, spirulin, capsaicin, propolis, ginseng, ginkgo biloba, coenzyme Q₁₀, alpha-lipoic acid, ω-3 unsaturated fatty acids, e.g. docosahexaenoic acid (DHA) and eicosapentaenoic acid (EPA), berry extracts such as bilberry, cranberry, currant and grape seed extracts, green tea extract, cactus, artichoke, papaya, melon, apple, hop, camellia, red clover, elderberry, rosemary, cocoa, olive leaf, pine bark and oyster extracts and other plant extracts containing polyphenols in a quantity greater than 1% by weight. Advantageously, said extracts may have previously undergone at least one fermentation step. In a preferred embodiment of the present invention, fermented papaya is used. Fermented papaya is produced and extracted from papaya fruits and from several tropical herbs following fermentation by yeast (preferably *Saccharomyces)* and bacteria. Fermented papaya enhances the activity of the strains of the present invention, not only as regards antioxidant activity, but also as regards the reduction in free radicals, inhibition of lipid peroxidation, reinforcement of the immune system, alkalinizing properties, and chelation of transition metals both in in vitro and in vivo experimental systems with consequent antiseptic action against microorganisms responsible for intestinal infections.

Said elements or substances with antioxidant activity as described above are added in a quantity by weight comprised from 0.0001% to 30% relative to the weight of the final composition, depending on the concentration of the substances with antioxidant activity and/or the recommended daily allowance (RDA), where defined.

Selenium can be present in the form of sodium selenate, sodium selenite and sodium acid selenite, as well as in the form of microorganisms, for example selenium-enriched yeast, in a quantity by weight comprised from 0.0005% to 0.005% relative to the weight of the final composition, in any case sufficient to contribute a quantity of selenium preferably comprised from 10 µg to 150 µg.

In a preferred embodiment, the composition of the present invention further comprises one or more bacterial strains capable of internalizing the selenium.

Strains that have application in particular are the strains deposited by the company BIOMAN S.r.l., Via Alfieri 18, 10100 Turin, namely: *Lactobacillus buchneri* LB26BM, deposited with the DSMZ on 05/04/2004 and having the deposit number DSM 16341; *Lactobacillus ferintoshensis* LB6BM, deposited with the DSMZ on 17/01/2004 and having the deposit number DSM 16144; *Lactobacillus reuteri* LB2BM, deposited with the DSMZ on 17/01/2004 and having the deposit number DSM 16143, in association with the strains with antioxidant activity of the present invention.

Said strains, in fact, are capable of accumulating inside cells large quantities of selenium, especially in organic form, if grown in the presence of a suitable source of selenium in the culture medium.

Glutathione is a strong antioxidant, surely one of the most important among those that the body is capable of producing. It has considerable action both against free radicals and molecules such as hydrogen peroxide, nitrites, nitrates, benzoates and others. It performs an important action in red blood cells, protecting said cells from dangerous oxidative stress which would cause haemolysis. In particular, the antioxidant form is referred to as reduced glutathione (or GSH).

In a preferred embodiment, the composition comprises glutathione in reduced form and selenium in a quantity by weight comprised from 0.5% to 10%, relative to the weight of the final composition.

Advantageously, since glutathione can be partially inactivated if taken orally, the composition can comprise the sulphur amino acid cysteine and/or N-acetylcysteine and/or mixtures thereof.

In a preferred embodiment, use is made of tomato seed oil, as it is particularly rich in lycopene, a carotenoid with marked antioxidant activity, in association with the antioxidant strains of the present invention.

In the composition of the present invention, the mixture of bacterial strains is present in a quantity comprised from 0.5 to 20% by weight, relative to the total weight of the composition, preferably from 2.5 to 8%.

In a preferred embodiment, the composition can further comprise at least one prebiotic fibre and/or carbohydrates having a bifidogenic action, such as, for example, inulin, fructo-oligosaccharides (FOS), galacto- and trans-galactooligosaccharides (GOS and TOS), gluco-oligosaccharides (GOSα), xylo-oligosaccharides (XOS), chitosan oligosaccharides (COS), soy oligosaccharides (SOS), isomalto-oligosaccharides (IMOS), resistant starch, pectin, psyllium, arabinogalactans, glucomannans, galactomannans, xylans, lactosucrose, lactulose, lactitol and various other types of gums, acacia, carob, oat or bamboo fibre, citrus fibres and, in general, fibres containing a soluble and an insoluble portion, in a variable ratio to each other.

In a preferred embodiment of the invention, the composition comprises at least one prebiotic fibre selected from among the above-mentioned ones and/or suitable mixtures thereof in any relative percentage whatsoever.

The quantity of the prebiotic fibres and/or of the carbohydrates having bifidogenic action, if present in the composition, is comprised from 0 to 60% by weight, preferably from 5 to 45% and even more preferably from 10 to 30%, relative to the total weight of the composition. In this case the composition or supplement has symbiotic activity and functional properties. Moreover, the internal part of the food product or supplement can also comprise other active ingredients and/or components, such as vitamins, minerals, bioactive peptides, substances having antioxidant, hypocholesterolemizing, hypoglycemizing, antiinflammatory activity, anti-sweetening agents in a quantity by weight generally comprised from 0.001% to 20% by weight, preferably from 0.01% to 5%, depending in any case on the type of active component and the recommended daily dose thereof, if defined, relative to the total weight of the composition.

The food composition of the present invention, for example a symbiotic composition, or else a supplement or a pharmaceutical composition, is prepared using techniques and apparatus known to a person skilled in the art.

In a preferred embodiment, the composition contains bacteria in a concentration comprised from 1x10⁶ to 1x10¹¹ CFU/g of mixture, preferably from 1x10⁸ to 1x10¹⁰ CFU/g of mixture.

In a preferred embodiment, the composition contains bacteria in a concentration comprised from 1x10⁶ to 1x10¹¹ CFU/dose, preferably from 10x10⁸ to 1x10¹⁰ CFU/dose.

The dose can be comprised from 0.2 to 10 g, for example it is 0.25 g, 1 g, 3 g, 5 g or 7 g.

The probiotic bacteria used in the present invention can be in solid form, in particular in powder, dehydrated powder or lyophilized form.

In a preferred embodiment, the mixture of bacterial strains comprises at least one strain selected from among *Bifidobacterium lactis* BS 05 (ID 1666) DSM 23032, *Lactobacillus acidophilus* LA 06 (ID 1683) DSM 23033 and *Lactobacillus brevis* LBR01 (ID 1685) DSM 23034 in microencapsulated form, i.e. said at least one strain (or all three said bacterial strains) is coated with a composition containing at least one lipid, preferably of vegetable origin. The microencapsulated bacteria are then added, using working methods known to a person skilled in the art, to an oil-based liquid composition so as to give an oily suspension.

The above-mentioned bacteria which are added to the oil-based liquid composition can be in the form of microencapsulated bacteria and/or "naked" non-microencapsulated bacteria, or mixtures thereof.

The bacteria selected from among *Bifidobacterium lactis* BS 05 (ID 1666) DSM 23032, *Lactobacillus acidophilus* LA 06 (ID 1683) DSM 23033 and *Lactobacillus brevis* LBR01 (ID 1685) DSM 23034, preferably in microencapsulated form, can be microencapsulated by means of common technologies known to a person skilled in the art. For example, a fluid bed technique (e.g. top spray or bottom spray) can be employed, in which coating materials of a lipid nature can be used.

In a preferred embodiment, saturated vegetable fats are used having a melting point below 75°C, preferably comprised from 45 to 65°C.

In a preferred embodiment, saturated vegetable fats having a certain degree of hydrophilicity can be used; these can be selected from among mono- and di-glycerides of saturated fatty acids, polyglycerols esterified with saturated fatty acids and free saturated fatty acids.

For example, polyglyceryl distearate (commercial name Plurol Stearique WL 1009), glyceryl palmitostearate (commercial name Precirol Ato 5), saturated fatty acids (commercial name Revel C) or hydrogenated vegetable fats of non-lauric origin can be used.

In a preferred embodiment, the ratio by weight between lyophilized microorganism and the lipid coating material which coats it is 50:50 or 40:60.

In a first embodiment, two lipids selected between a hydrogenated palm fat (Tm=60°C) and glycerol dipalmitostearate (Tm=57-60°C) are sprayed onto the lyophilizate in succession, i.e. a double covering is applied to the lyophilizate: the first with the hydrogenated palm fat and the second with the glycerol dipalmitostearate in a ratio of 3:1 to each other. A double coating of the cells ensures better sealing of the bacteria from the environment, producing a continuous film without pores communicating with the outside. However, this wrapper must open at the intestinal level to release the bacteria and allow them to colonise. The selected lipids are in fact resistant to acid pH's, so that the coating remains intact in the stomach, but sensitive to even slightly basic pH's, so as to allow the formation of holes in the coating during their passage through the intestine.

In a preferred embodiment, the composition of the present invention is an oil-based composition comprising lactic bacteria coated as mentioned above. Said composition is prepared according to techniques known to a person skilled in the art.

In practical terms, a given quantity of oil is introduced into a container provided with stirring and heating means. Subsequently the coated probiotic bacteria in solid form are gradually added under stirring so as to avoid the formation of lumps and agglomerates. Once the addition of bacteria has ended, the oily suspension is maintained under stirring for a time comprised from 1 to 30 minutes, if necessary with slight heating to a temperature comprised from 25 to 40°C, preferably from 30 to 35°C.

The composition that is obtained is similar to an oily suspension. The composition contains the bacteria in a quantity less than or equal to 30% by weight, comprised from 0.05 to 20% by weight, relative to the total weight of the composition; preferably in a quantity comprised from 0.5 to 10%; even more preferably in a quantity comprised from 1.5 to 5% by weight, relative to the total weight of the composition.

The composition comprises at least one edible oil suitable to be administered to subjects in paediatric age, selected from the group comprising: olive oil, maize oil, soybean oil, linseed oil, peanut oil, sesame oil, fish oil and rice oil and other seed oils among which, in particular, tomato seed oil can be used. Said oils can be used individually or together in suitable mixtures, in appropriate weighted ratios. Advantageously, said oils are of biological grade and their preparation can include a refinement step and/or a cold pressing step.

The composition comprises at least one oil in a quantity greater than or equal to 70% by weight, relative to the total weight of the suspension, preferably in a quantity comprised from 75 to 95% by weight, advantageously at least 90% by weight. Advantageously, the composition contains only olive oil or else olive oil in a mixture with maize oil and/or soybean oil and/or linseed oil and/or tomato seed oil. Advantageously, the olive oil is extra-virgin and of Bio grade.

In a preferred embodiment, the composition further comprises at least one finely divided food compound selected from the group comprising silica, silicon dioxide, silica gel, colloidal silica, precipitated silica, Syloid^{®} 244, talc, magnesium silicate, magnesium oxide, magnesium carbonate, calcium silicate, lecithin, mono- or di-glycerides such as glyceryl monostearate, glyceryl monooleate, plurol-oleic acid, starch, modified starches, konjac gum, xanthan gum, gellan gum and carrageenan.

Said material is present in a quantity comprised from 0.1 to 15% by weight, relative to the total weight of the composition, preferably from 1 to 5% by weight, relative to the total weight of the composition.

In this case the preparation procedure provides that, to a given quantity of oil, the finely divided food material, for example silicon dioxide, is added under stirring. Subsequently, the oil containing said material is heated under stirring at around 60°C until complete dissolution.

Alternatively, the silicon dioxide can also be added cold; however, solubilisation requires a longer time. Subsequently, the composition is allowed to cool from 60°C to room temperature. Then the lyophilizate is weighed and added to the suspension under stirring, until completely and homogeneously dispersed. The composition that is obtained is similar to an oily suspension.

Examples of preferred compositions, in accordance with the present invention, are shown in Table 3 (Examples 1-4).

Examples 1-4 are given solely by way of non-restrictive example of the present invention and consider a volume of oily suspension suitable for a treatment period equal to 30 days. The viable count shown, expressed in billions of living cells, thus refers to 30 doses. A single dose is capable of providing, at the time of manufacture, 1.5 billion/strain in examples 1 and 2 and 2.5 billion/strain in examples 3 and 4.

The Applicant has found it possible to use different volumes of oily suspension, for example 5 ml, suitable for shorter treatment periods.

In a preferred embodiment, the oily suspension can further comprise, in a quantity comprised from 0.5 to 25% by weight, relative to the total weight of the suspension, at least one prebiotic fibre and/or at least one bifidogenic carbohydrate selected from among inulin, fructo-oligosaccharides (FOS), galacto- and trans-galacto-oligosaccharides (GOS and TOS), gluco-oligosaccharides (GOSα), xylo-oligosaccharides (XOS), chitosan oligosaccharides (COS), soy oligosaccharides (SOS), isomalto-oligosaccharides (IMOS), maltodextrin, resistant starch, pectin, psyllium, arabinogalactans, glucomannans, galactomannans, xylans, lactosucrose, lactulose, lactitol, acacia fibre, carob fibre, oat fibre, bamboo fibre and citrus fibre.

The prebiotic fibres and the carbohydrates have a dual function. The first is that of performing a prebiotic effect. The second is that of performing a technological effect as a thickener and stabilizer. Advantageously, said at least one fibre and said at least one carbohydrate are selected from among gluco-oligosaccharides (GOSα), fructo-oligosaccharides (FOS), inulin and/or maltodextrin. The suspension contains strains of microencapsulated microorganisms with at least one lipid having a melting point below 75°C, preferably comprised from 45 to 65°C.

The suspension is indicated for use as a medication for the treatment of intestinal disorders, such as, for example, colic in paediatric subjects.

In another preferred embodiment, the composition of the present invention is formulated in sachets. Table 4 shows examples 5-8.

In example 6, the strain *Lactobacillus buchneri* LB26BM (DSM 16341) contains 50 µg/g of selenium accumulated inside the cell prevalently in the form of selenium methionine and selenium cysteine; therefore, 1 gram is capable of providing 90% of the RDA of said element.

In example 7, the composition comprises 3 grams of fermented papaya having action synergistic with the strain *B. lactis* BS05 (DSM 23032).

Below is a description of the operating conditions for cultivating the strains *Bifidobacterium lactis* BS 05 (ID 1666) DSM 23032, *Lactobacillus acidophilus* LA 06 (ID 1683) DSM 23033 and *Lactobacillus brevis* LBR01 (ID 1685) DSM 23034. The conditions are valid for all strains unless otherwise indicated.
i) Medium used: TPY broth + Cys HCl 0.5 g/l for DSM 23032, and Difco MRS ref. 288130 for DSM 23033 and DSM 23034.
ii) pH of the medium prior to sterilization: 7.10 for DSM 23032 and 7.00 for DSM 23033 and DSM 23034.
iii) Sterilization: 15 minutes at 121°C.
iv) pH of the medium after sterilization: 6.60 for DSM 23032 and 6.50 for DSM 23033 and DSM 23034.
v) Relationship with oxygen: anaerobic species obligatory for DSM 23032 and optional anaerobic or microaerophilic species for DSM 23033 and DSM 23034.
vi) Incubation temperature: 37°C.
vii) Incubation time: 17 hours for DSM 23032 and 15 hours for DSM 23033 and DSM 23034.
viii) Short-term storage temperature: 5°C.
ix) Transfer time: 2 days.
x) Long-term storage temperature: -25°C.
xi) Conditions for testing viability: growth in TPY broth at 37°C overnight or until adequate turbidity is reached for DSM 23032 and growth in MRS broth at 37°C overnight for DSM 23033 and DSM 23034.
xii) Description: rods of varying shapes, gram positive, non-rapid formation of acidity, no spore formation, anaerobic, degrades glucose exclusively and in a characteristic manner via the fructose-6-phosphate phosphoketolase route in the case of DSM 23032; rods with rounded ends, arranged singly or in chains, good growth at 37°C and homofermentative metabolism obligatory for DSM 23033, heterofermentative obligatory for DSM 23034.

### Experimental part

The Applicant carried out a large-scale screening of numerous probiotic bacteria with the aim of identifying one or more microorganisms endowed with antioxidant activity.

The first screening activity was carried out through a series of in vitro tests. In particular, the total antioxidant activity (TAA) both of whole cells and cell extracts was evaluated.

In the case of whole cells, the potential antioxidant activity was investigated by means of two tests:
- Autooxidation of ascorbic acid AA%
- Oxidation of linolenic acid LA%

Both tests quantify the ability of the bacterial strain, used as whole cells, to protect ascorbic acid or linolenic acid from oxidation.

In detail, the kinetics of the autooxidation reaction of ascorbic acid can be determined by spectrophotometrically recording at 265 nm the presence of dehydroascorbic acid, thus offering a measure of the antioxidant power, assessed as the capacity to inhibit said autooxidation reaction.

A thiobarbituric acid assay was instead used to monitor the capacity of the bacterial strains to inhibit the peroxidation of linolenic acid. The oxidation of linolenic acid causes, in fact, an autocatalytic chain that leads to the formation of different radical species. One of the products of decomposition of radical species is malonaldehyde, which can be used as an indicator of oxidative stress in the thiobarbituric acid assay, since it is capable of reacting with said acid to form a red chromogenic complex which can be spectrophotometrically determined at 534 nm.

The research work conducted on cell extracts instead included the following tests:
- Trolox^{®} Equivalence Antioxidant Capacity (TEAC) %
- Glutathione (GSH) nmoles/mg
- Superoxide dismutase (SOD) U/mg

The first test is based on the reaction of antioxidant molecules with the cationic radical ABTS●+ (2,2'-Azinobis(3-ethylbenzothiazoline-6-sulfonate)).

This radical can be reduced, with a consequent loss of absorbance, from an antioxidant whose scavenger capacity can be spectrophotometrically measured at 734 nm. The test is performed at 37°C and the results are obtained by comparison with Trolox^{®} (synthetic antioxidant, a hydrophilous vitamin E analogue), so as to define the millimolar concentration of a Trolox^{®} solution which has an antioxidant capacity equivalent to that of a 1 mM solution of the substance under analysis (TEAC).

The second and the third test measure the concentration of reduced glutathione (GSH) and of the enzyme superoxide dismutase (SOD) that the single bacterial strain is capable of producing. Both molecules are known to have antioxidant activity.

The activity of superoxide dismutase (SOD) is determined by means of the spectrophotometric method, exploiting the principle of enzyme inhibition of the oxidation of epinephrine (4-[1-hydroxy-2-(methylamino)-ethyl]-1,2- benzenediol). Oxidation by 02 occurs at an alkaline pH with the production of superoxide anions (02-) which, by accumulating in the solution, promote the conversion of epinephrine to adrenochrome (3-hydroxy-1-methyl-5,6-indolindion), a coloured compound which allows the course of the reaction to be monitored, based on a measurement of absorbance.

The presence of superoxide dismutase (SOD) removes 02-ions and reduces the speed of formation and the quantity of adrenochrome. The percentage of inhibition of oxidation is a hyperbolic function of the SOD concentration.

A quantitative analysis of glutathione can be performed using a spectrophotometric method that relies on Ellman's enzymatic reaction: the sulphidrilic group of glutathione (GSH) reacts with 5,5'-dithiobis-2-nitrobenzoic acid (DTNB), forming a yellow-coloured compound: 5-thio-2-nitrobenzoic acid (TNB). Simultaneously, the oxidized glutathione is again reduced by glutathione reductase, leading to further formation of TNB. The rate of formation of TNB is directly proportional to the total GSH concentration, expressed as nanomoles per mg of protein present in the cell extract.

The results are shown in Table 1.

From Table 1 it may be inferred that the strains possess a significant antioxidant activity, observed both for whole cells and cell extracts.

Subsequently, to confirm the antioxidant activity of the strains of the present invention also in an in vivo model, the Applicant conducted an animal study.

In particular, the study was conducted on a sample population of 48 healthy adult Wistar rats (Harlan, Milano, Italy), which were fed a standard diet for 7 days.

Subsequently, the 48 rats were divided into the following groups:
- Group C: control group comprising 20 rats fed only the standard diet, without probiotics, for 18 days.
- Group T1: study group comprising 7 rats fed a diet, for 18 days, supplemented with a mixture of bacteria containing the strains *Bifidobacterium lactis* BS 05 (ID 1666) DSM 23032, *Lactobacillus acidophilus* LA 06 (ID 1683) DSM 23033 and *Lactobacillus brevis* LBR01 (ID 1685) DSM 23034 in a weighted ratio of 1:1:1 (hereinafter indicated as M for the sake of brevity) at a concentration of 1x10⁹ CFU/day. In detail, the aforesaid probiotic strains were mixed with the feed of the rats in a concentration such that a total of 1x10⁹ CFU was present in the average quantity consumed daily by a single animal.
- Group T2: study group comprising 14 rats fed a diet supplemented with M mixture, as described above, at a concentration of 1x10⁸ CFU/day for 18 days.
- Group T3: study group comprising 7 rats fed a diet supplemented with M mixture, as described above, at a concentration of 1x10⁷ CFU/day for 18 days.

At the end of the 18 days the rats in group C and group T2 were divided into two subgroups of respectively 10 and 7 rats each: one subgroup (Cf and T2f) was injected with a saline solution and the other with a solution of Doxorubicin (abbreviated DOX), an antineoplastic drug with pro-oxidative activity which is capable of inducing strong oxidative stress, at a dose of 20 µg/g of body weight (CDOX and T2DOX). All rats of groups T1 and T3 were injected with the same dose of Doxorubicin (T1DOX and T3DOX).

The rats were then sacrificed within 24 hours after injection of the saline solution or DOX. Plasma samples were then taken in order to quantify the total antioxidant activity (TAA) in vitro and the levels of glutathione in reduced form (GSH) (Figures 1 and 2). Comparing CDOX and T1DOX, T2DOX and T3DOX enables us to evaluate a possible protection on the part of the mixture of probiotics against the oxidative stress induced by Doxorubicin, also revealing a possible dose-response relationship according to the number of viable cells administered.

The comparison between Cf and T2f serves to reveal a possible increase in antioxidant activity induced by the probiotic strains under physiological conditions, in the absence, therefore, of the oxidative stress induced by the Doxorubicin.

In Fig. 1 and 2 (total antioxidant activity, TAA and plasma glutathione, respectively) the effect of the treatment with probiotics in the case of oxidative stress (differences between the control group CDOX and the groups T1DOX, T2DOX and T3DOX) were evaluated by means of ANOVA statistical analysis, comparing all the rats that had received the DOX. The effects of the administration of Doxorubicin were evaluated by means of the ANOVA test (using the Tukey post test) and by comparing all the groups treated with DOX with the baseline control (Cf). The differences between the control (Cf) and the group treated with probiotics under baseline conditions (T2f) were evaluated with the Student's t test.

From figure 1 it may be observed that, under baseline conditions (C and T2), treatment with probiotics does not modify the TAA (non-significant comparison, n.s.). In the rats not treated with probiotics, the administration of DOX causes a significant decline in TAA compared to baseline values (t test p<0.001). In the rats treated with probiotics as well, the administration of DOX causes a decrease in TAA compared to baseline values (Cf), but in the treatments at a higher concentration of probiotics (T1 and T2) the decrease is smaller (t test p<0.02). In rats treated with a lower concentration of probiotics (T3), after the administration of DOX the decrease in TAA is greater (t test p<0.001). The difference among the groups treated with DOX is significant (ANOVA p<0.05). In figure 2, the concentration of GSH is used as a measure of antioxidant capacity. Under oxidative stress the levels of GSH are lowered.

C vs. T2: t test p>0.05 (not statistically significant, n.s.) ANOVA test comparing C with all the groups treated with DOX: p<0.001.

Post test (Tukey):
C vs. C+DOX p<0.001
C vs. T1+DOX n.s.
C vs. T2+DOX p<0.05
C vs. T3+DOX p<0.01

Figure 2 shows that under baseline conditions there is no difference between controls and rats treated with the M mixture. In the control rats, oxidative stress causes a strong decrease in the concentration of GSH (p<0.001). In rats treated with the M mixture at the highest concentration (T1), the injection of DOX does not cause a significant reduction in the levels of GSH (n.s.). In the rats treated with an intermediate dose of the M mixture, a decrease is observed compared to the baseline control, but the statistical significance is lower (p<0.05). In the rats treated with probiotics at the smallest dose the decrease in GSH levels is even more evident (p<0.01). The difference among the various groups treated with DOX is significant (ANOVA p<0.05). All the data reported above show that the administration of a mixture of the three probiotic strains (M mixture) is capable of reducing the oxidative stress induced by the injection of Doxorubicin, an antineoplastic drug with pro-oxidant activity (see figure 1, comparison between C + DOX and T1 + DOX).

The oxidative stress was evaluated both in terms of the decrease in total antioxidant activity at the plasma level and that of glutathione in reduced form (GSH). Glutathione is an important molecule capable of combating oxidative stress and of neutralising the free radicals present in plasma.

The most interesting comparisons are between group C + DOX (control group, treated without probiotics and with injection of Doxorubicin after 18 days) and groups T1 + DOX/T2 + DOX (groups treated with probiotics, respectively an amount of 10⁹ and 10⁸ CFU/day, which were injected with Doxorubicin after 18 days). The lowest concentration of probiotics, i.e. 10⁷ CFU/day (group T3), showed to be of more limited utility in reducing the oxidative stress induced by the Doxorubicin.

In any case we believe that the concentration of 10⁸ CFU/day in rats may be representative of the quantities habitually used in humans (10¹⁰-10¹¹ CFU/day).

## Claims

1. At least one bacterial strain having antioxidant properties belonging to a species selected from among:
- *Bifidobacterium lactis* BS 05 (ID 1666) deposited by Probiotical SpA, Novara (Italy) with the DSMZ in Germany on 13.10.2009 and having the deposit number DSM 23032,
- *Lactobacillus acidophilus* LA 06 (ID 1683) deposited by Probiotical SpA, Novara (Italy) with the DSMZ in Germany on 13.10.2009 and having the deposit number DSM 23033, and
- *Lactobacillus brevis* LBR01 (ID 1685) deposited by Probiotical SpA, Novara (Italy) with the DSMZ in Germany on 13.10.2009 and having the deposit number DSM 23034 for use in the treatment of oxidative stress.

2. At least one bacterial strain for the use according to claim 1, wherein said oxidative stress has been induced as a result of the intake of drugs.

3. A composition comprising a mixture containing at least one bacterial strain having antioxidant properties selected from among:
- *Bifidobacterium lactis* BS 05 (ID 1666) deposited by Probiotical SpA, Novara (Italy) with the DSMZ in Germany on 13.10.2009 and having the deposit number DSM 23032,
- *Lactobacillus acidophilus* LA 06 (ID 1683) deposited by Probiotical SpA, Novara (Italy) with the DSMZ in Germany on 13.10.2009 and having the deposit number DSM 23033, and
- *Lactobacillus brevis* LBR01 (ID 1685) deposited by Probiotical SpA, Novara (Italy) with the DSMZ in Germany on 13.10.2009 and having the deposit number DSM 23034, for use in the treatment of oxidative stress.

4. The composition for use according to claim 3, wherein the oxidative stress has been induced as a result of the intake of drugs.

5. The composition for use according to claim 3, wherein the composition is a food composition or supplement.

6. The composition for use according to any one of claims 3-5, wherein said composition contains bacteria in a concentration comprised from 1x10⁶ to 1x10¹¹ CFU/g of mixture.

7. The composition for use according to any one of claims 3-6, wherein said composition contains bacteria in a concentration comprised from 1x10⁸ to 1x10¹⁰ CFU/g of mixture.

8. The composition for use according to any one of claims 3-7, wherein said composition contains bacteria in a concentration comprised from 1x10⁶ to 1x10¹¹ CFU/dose.

9. The composition for use according to any one of claims 3-8, wherein said composition contains bacteria in a concentration comprised from 1x10⁸ to 1x10¹⁰ CFU/dose.

10. The composition for use according to any one of claims 1-9, wherein said mixture comprises bacteria coated with at least one saturated vegetable fat having a melting point below 75°C.

11. The composition for use according to any one of claims 1-10, wherein said mixture comprises bacteria coated with at least one saturated vegetable fat having a melting point comprised from 45 to 65°C.

12. The composition for use according to any one of claims 10-11, wherein the at least one saturated vegetable fat is selected from among mono- and di-glycerides of saturated fatty acids, polyglycerols esterified with saturated fatty acids and free saturated fatty acids.

13. The composition for use according to any one of claims 10-12, wherein said coated bacteria are added to an oil of vegetable origin selected from the group comprising: olive oil, maize oil, soybean oil, linseed oil, peanut oil, sesame oil, fish oil and rice oil and other seed oils among which, in particular, tomato seed oil can be used.

14. The composition for use according to any one of claims 3-13, wherein said composition further comprises elements or substances having antioxidant activity, such as, for example, selenium, zinc, magnesium, manganese, glutathione, superoxide dismutase (SOD), vitamin C, vitamin E, beta-carotene, lycopene, tomato seed oil, quercetin, tyrosol, resveratrol, hydroxytyrosol, oleuropein, lutein, spirulin, alpha-lipoic acid, ω-3 unsaturated fatty acids, e.g. docosahexaenoic acid (DHA) and eicosapentaenoic acid (EPA), berry extracts such as bilberry, cranberry, currant and grape seed extracts, green tea extract, cactus, artichoke, papaya, melon, apple, hop, camellia, red clover, elderberry, rosemary, cocoa, olive leaf, pine bark and oyster extracts and other plant extracts containing polyphenols in an amount greater than 1% by weight and papaya.

15. The composition for use according to claim 14, wherein elements or substances having antioxidant activity are extracts that have previously undergone fermentation.

## Patentansprüche

1. Wenigstens ein Bakterienstamm mit antioxidativen Eigenschaften, der zu einer Art, ausgewählt aus:
- *Bifidobacterium lactis* BS 05 (ID 1666), hinterlegt durch Probiotical SpA, Novara (Italien) am 13.10.2009 bei der DSMZ in Deutschland mit der Hinterlegungsnummer DSM 23032,
- *Lactobacillus acidophilus* LA 06 (ID 1683), hinterlegt durch Probiotical SpA, Novara (Italien) am 13.10.2009 bei der DSMZ in Deutschland mit der Hinterlegungsnummer DSM 23033, und
- *Lactobacillus brevis* LBR01 (ID 1685), hinterlegt durch Probiotical SpA, Novara (Italien) am 13.10.2009 bei der DSMZ in Deutschland mit der Hinterlegungsnummer DSM 23034,
gehört,zur Verwendung bei der Behandlung von oxidativem Stress.

2. Wenigstens ein Bakterienstamm zur Verwendung gemäß Anspruch 1, wobei der oxidative Stress infolge der Einnahme von Medikamenten induziert wurde.

3. Zusammensetzung, umfassend eine Mischung, die wenigstens einen Bakterienstamm mit antioxidativen Eigenschaften enthält, der aus
- *Bifidobacterium lactis* BS 05 (ID 1666), hinterlegt durch Probiotical SpA, Novara (Italien) am 13.10.2009 und mit der Hinterlegungsnummer DSM 23032 bei der DSMZ in Deutschland,
- *Lactobacillus acidophilus* LA 06 (ID 1683), hinterlegt durch Probiotical SpA, Novara (Italien) am 13.10.2009 und mit der Hinterlegungsnummer DSM 23033 bei der DSMZ in Deutschland, und
- *Lactobacillus brevis* LBR01 (ID 1685), hinterlegt durch Probiotical SpA, Novara (Italien) am 13.10.2009 und mit der Hinterlegungsnummer DSM 23034 bei der DSMZ in Deutschland,
ausgewählt wird, zur Verwendung bei der Behandlung von oxidativem Stress.

4. Zusammensetzung zur Verwendung gemäß Anspruch 3, wobei der oxidative Stress infolge der Einnahme von Medikamenten induziert wurde.

5. Zusammensetzung zur Verwendung gemäß Anspruch 3, wobei die Zusammensetzung ein(e) Lebensmittelzusammensetzung oder -ergänzungsmittel ist.

6. Zusammensetzung zur Verwendung gemäß einem der Ansprüche 3 bis 5, wobei die Zusammensetzung Bakterien in einer Konzentration von 1 x 10⁶ bis 1 x 10¹¹ KBE/g Mischung enthält.

7. Zusammensetzung zur Verwendung gemäß einem der Ansprüche 3 bis 6, wobei die Zusammensetzung Bakterien in einer Konzentration von 1 x 10⁸ bis 1 x 10¹⁰ KBE/g Mischung enthält.

8. Zusammensetzung zur Verwendung gemäß einem der Ansprüche 3 bis 7, wobei die Zusammensetzung Bakterien in einer Konzentration von 1 x 10⁶ bis 1 x 10¹¹ KBE/Dosis enthält.

9. Zusammensetzung zur Verwendung gemäß einem der Ansprüche 3 bis 8, wobei die Zusammensetzung Bakterien in einer Konzentration von 1 x 10⁸ bis 1 x 10¹⁰ KBE/Dosis enthält.

10. Zusammensetzung zur Verwendung gemäß einem der Ansprüche 1 bis 9, wobei die Mischung Bakterien, beschichtet mit wenigstens einem gesättigten pflanzlichen Fett mit einem Schmelzpunkt unterhalb von 75°C, umfasst.

11. Zusammensetzung zur Verwendung gemäß einem der Ansprüche 1 bis 10, wobei die Mischung Bakterien, beschichtet mit wenigstens einem gesättigten pflanzlichen Fett mit einem Schmelzpunkt von 45 bis 65°C, umfasst.

12. Zusammensetzung zur Verwendung gemäß einem der Ansprüche 10 bis 11, wobei das wenigstens eine gesättigte pflanzliche Fett aus Mono- und Diglyceriden gesättigter Fettsäuren, mit gesättigten Fettsäuren veresterten Polyglycerolen und freien gesättigten Fettsäuren ausgewählt wird.

13. Zusammensetzung zur Verwendung gemäß einem der Ansprüche 10 bis 12, wobei die beschichteten Bakterien zu einem Öl pflanzlichen Ursprungs, ausgewählt aus der Gruppe, umfassend: Olivenöl, Maisöl, Sojabohnenöl, Leinsamenöl, Erdnussöl, Sesamöl, Fischöl und Reisöl und andere Kernöle, unter welchen insbesondere Tomatenkernöl verwendet werden kann, zugegeben werden.

14. Zusammensetzung zur Verwendung gemäß einem der Ansprüche 3 bis 13, wobei die Zusammensetzung ferner Elemente oder Substanzen mit antioxidativer Aktivität umfasst, beispielsweise Selen, Zink, Magnesium, Mangan, Glutathion, Superoxiddismutase (SOD), Vitamin C, Vitamin E, Beta-Carotin, Lycopen, Tomatenkernöl, Quercetin, Tyrosol, Resveratrol, Hydroxytyrosol, Oleuropein, Lutein, Spirulin, Alpha-Liponsäure, ω-3-ungesättigte Fettsäuren, beispielsweise Docosahexaensäure (DHA) und Eicosapentaensäure (EPA), Beerenextrakte, beispielsweise Heidelbeer-, Moosbeer-, Johannisbeer- und Traubenkern-Extrakte, Grüntee-Extrakt, Kaktus-, Artischocke-, Papaya-, Melone-, Apfel-, Hopfen-, Kamelie-, Rotklee-, Holunderbeer-, Rosmarin-, Kakao-, Olivenblatt-, Kieferrinde- und Austern-Extrakte und weitere Pflanzen-Extrakte, enthaltend Polyphenole in einer Menge größer als 1 Gew.% und Papaya.

15. Zusammensetzung zur Verwendung gemäß Anspruch 14, wobei Elemente oder Substanzen mit anioxidativer Aktivität Extrakte, die vorher einer Fermentation unterzogen wurden, sind.

## Revendications

1. Au moins une souche bactérienne ayant des propriétés antioxydantes appartenant à une espèce choisie parmi :
*Bifidobacterium lactis* BS 05 (ID 1666) déposée par Probiotical SpA, Novara (Italie) avec la DSMZ en Allemagne le 13.10.2009 et ayant le numéro de dépôt DSM 23032,
*Lactobacillus acidophilus* LA 06 (ID 1683) déposée par Probiotical SpA, Novara (Italie) avec la DSMZ en Allemagne le 13.10.2009 et ayant le numéro de dépôt DSM 23033, et
*Lactobacillus brevis* LBR01 (ID 1685) déposée par Probiotical SpA, Novara (Italie) avec la DSMZ en Allemagne le 13.10.2009 et ayant le numéro de dépôt DSM 23034 pour une utilisation dans le traitement du stress oxydatif.

2. Au moins une souche bactérienne pour l'utilisation selon la revendication 1, dans laquelle le stress oxydatif a été induit à la suite de la prise de médicaments.

3. Composition comprenant un mélange contenant au moins une souche bactérienne ayant des propriétés antioxydantes, choisie parmi :
*Bifidobacterium lactis* BS 05 (ID 1666) déposée par Probiotical SpA, Novara (Italie) avec la DSMZ en Allemagne le 13.10.2009 et ayant le numéro de dépôt DSM 23032,
*Lactobacillus acidophilus* LA 06 (ID 1683) déposée par Probiotical SpA, Novara (Italie) avec la DSMZ en Allemagne le 13.10.2009 et ayant le numéro de dépôt DSM 23033, et
*Lactobacillus brevis* LBR01 (ID 1685) déposée par Probiotical SpA, Novara (Italie) avec la DSMZ en Allemagne le 13.10.2009 et ayant le numéro de dépôt DSM 23034,
pour une utilisation dans le traitement du stress oxydatif.

4. Composition pour une utilisation selon la revendication 3, dans laquelle le stress oxydatif a été induit à la suite de la prise de médicaments.

5. Composition pour une utilisation selon la revendication 3, dans laquelle la composition est un supplément ou une composition alimentaire.

6. Composition pour une utilisation selon l'une quelconque des revendications 3 à 5, dans laquelle ladite composition contient des bactéries à une concentration comprise entre 1x10⁶ et 1x10¹¹ UFC/g de mélange.

7. Composition pour une utilisation selon l'une quelconque des revendications 3 à 6, dans laquelle ladite composition contient des bactéries à une concentration comprise entre 1x10⁸ et 1x10¹⁰ UFC/g de mélange.

8. Composition pour une utilisation selon l'une quelconque des revendications 3 à 7, dans laquelle ladite composition contient des bactéries à une concentration comprise entre 1x10⁶ et 1x10¹¹ UFC/dose.

9. Composition pour une utilisation selon l'une quelconque des revendications 3 à 8, dans laquelle ladite composition contient des bactéries à une concentration comprise entre 1x10⁸ et 1x10¹⁰ UFC/dose.

10. Composition pour une utilisation selon l'une quelconque des revendications 1 à 9, dans laquelle ledit mélange comprend des bactéries recouvertes d'au moins une matière grasse végétale saturée ayant un point de fusion inférieur à 75°C.

11. Composition pour une utilisation selon l'une quelconque des revendications 1 à 10, dans laquelle ledit mélange comprend des bactéries recouvertes d'au moins une matière grasse végétale saturée ayant un point de fusion compris entre 45 et 65°C.

12. Composition pour une utilisation selon l'une quelconque des revendications 10 à 11, dans laquelle l'au moins une matière grasse végétale saturée est choisie parmi des mono- et diglycérides d'acides gras saturés, des polyglycérols estérifiés avec des acides gras saturés et des acides gras saturés libres.

13. Composition pour une utilisation selon l'une quelconque des revendications 10 à 12, dans laquelle lesdites bactéries recouvertes sont ajoutées à une huile d'origine végétale choisie dans le groupe comprenant : l'huile d'olive, l'huile de maïs, l'huile de soja, l'huile de lin, l'huile d'arachide, l'huile de sésame, l'huile de poisson et l'huile de riz et d'autres huiles de graines parmi lesquelles, en particulier, l'huile de pépins de tomate peut être utilisée.

14. Composition pour une utilisation selon l'une quelconque des revendications 3 à 13, dans laquelle ladite composition comprend en outre des éléments ou des substances ayant une activité antioxydante, tels/telles que, par exemple, le sélénium, le zinc, le magnésium, le manganèse, le glutathion, la superoxyde dismutase (SOD), la vitamine C, la vitamine E, le bêta-carotène, le lycopène, l'huile de pépins de tomate, la quercétine, le tyrosol, le resvératrol, l'hydroxytyrosol, l'oleuropéine, la lutéine, la spiruline, l'acide alpha-lipoïque, des acides gras insaturés ω-3, par exemple, l'acide docosahexaénoïque (DHA) et l'acide eicosapentaénoïque (EPA), des extraits de baies tels que des extraits de myrtille, de canneberge, de groseille et de pépins de raisin, un extrait de thé vert, des extraits de cactus, d'artichaut, de papaye, de melon, de pomme, de houblon, de camélia, de trèfle rouge, de sureau, de romarin, de cacao, de feuille d'olivier, d'écorce de pin et d'huître et d'autres extraits de plantes contenant des polyphénols en une quantité supérieure à 1% en poids et la papaye.

15. Composition pour une utilisation selon la revendication 14, dans laquelle des éléments ou des substances ayant une activité antioxydante sont des extraits qui ont précédemment subi une fermentation.
